# EUROPÄISCHE PATENTSCHRIFT

(11) **EP 2 359 892 B1**
(45) Veröffentlichungstag und Bekanntmachung des Hinweises auf die Patenterteilung: **10.10.2012**
(21) Anmeldenummer: 11151816.3
(22) Anmeldetag: 24.01.2011
(51) Int. Cl.: A61M 25/10

(54) **Ballonkatheter**
Balloon catheter
Cathéter à ballonnet

(30) Priorität: 15.02.2010 US 304489 P
(43) Veröffentlichungstag der Anmeldung: 24.08.2011
(73) Patentinhaber: Biotronik AG, 8180 Bülach (CH)
(72) Erfinder: Wesselmann, Matthias, 8912, Glattfelden (CH); Lang, Hans, 8107, Buchs (CH); Pfenninger-Ganz, Susanne, 8607, Aathal (CH); Blaser, Adrian, 8032, Zürich (CH); Hoser, Christoph Hannes, 8008, Zürich (CH); Fischer, Horst Josef, 79787, Lauchingen (DE); Quint, Bodo, 8154, Oberglatt (CH)
(74) Vertreter: Lindner-Vogt, Karin L.

(56) Entgegenhaltungen:
- EP-A1- 2 149 387
- GB-A- 2 046 096
- US-A- 5 108 525

## Beschreibung

Die Erfindung betrifft einen Ballonkatheter gemäß dem Oberbegriff des Anspruchs 1.

Im Oberbegriff des Anspruches 1 ist die Grundkonstruktion von Ballonkathetem angegeben, wie sie beispielsweise aus den Druckschriften US 5,522,882 A und US 7,217,278 B2 bekannt ist. So weisen Ballonkatheter, wie sie etwa für das Aufweiten von krankhaft verengten Gefäßen im Körper oder für das Setzen von Gefäßwandstützen - sogenannter "Stents" - eingesetzt werden, einen Außenschaft mit einem distalen Ende und einen darin unter Bildung einer ringförmigen Fluidleitung angeordneten Innenschaft auf, der über das distale Ende des Außenschaftes hinaussteht. Am distalen Ende des Katheters ist ein Ballon mit einer ersten Befestigungszone an seinem proximalen Ende fluiddicht auf dem distalen Endbereich des Außenschaftes und mit einer zweiten Befestigungszone an seinem distalen Ende fluiddicht auf dem distalen Endbereich eines durch den Innenschaft gebildeten Innenteils befestigt. Zwischen diesen Befestigungszonen ist der Ballon in undilatiertem Zustand in Längsfalten gelegt, damit sein Außendurchmesser in diesem Zustand möglichst gering ist. Dies ist Voraussetzung dafür, dass der Ballonkatheter mit dem Ballon am distalen Ende auch durch enge Gefäße oder stark gekrümmte Gefäßbereiche vorgeschoben werden kann. Nach der Platzierung des Ballons an seinem Einsatzort kann dann über die zwischen Innen- und Außenschaft gebildete ringförmige Fluidleitung ein Fluid unter Druck eingebracht und der Ballon dilatiert werden. Dabei entfalten sich die Längsfalten in Peripherrichtung unter starker Durchmesservergrößerung des Ballons.

Die Ballons herkömmlicher Ballonkatheter weisen aufgrund ihrer Herstellungsart und Konstruktion Nachteile auf, die aus dem folgenden Abriss des Produktionsprozesses deutlich werden. So werden Ballons in der Regel aus einem plastisch reckbaren Kunststoffröhrchen mit einem Außendurchmesser von beispielsweise 2,1 mm und einem Lumen-Durchmesser von beispielsweise 1,5 mm hergestellt. Die Wandstärke dieses Röhrchens beträgt also 0,3 mm. Seine Enden werden in eine Haltevorrichtung eingespannt, worauf das Lumen mit einem Fluiddruck beaufschlagt wird. Zwischen den Einspannpunkten wird das Werkstück aufgeblasen und das Wandmaterial drastisch gereckt, so dass ein im Wesentlichen zylindrischer Ballon mit einer Wandstärke beispielsweise 0,03 mm entsteht. Von den eingespannten Enden des Ballonrohlings aus verringert sich die Wandstärke über die Konen an den beiden Enden des Ballons zur Mantelwand hin also etwa um den Faktor 10.

In einem weiteren Bearbeitungsschritt werden die Enden noch kalibriert und beispielsweise auf einen Außendurchmesser von 1,8 mm sowie einen Lumen-Durchmesser von 1,6 mm gebracht. Die Wandstärke beträgt dann noch 0,1 mm und beträgt somit immer noch mehr als das Dreifache der Wandstärke im zylindrischen Teil des Ballonrohlings.

Werden nun derart hergestellte Ballons mit ihren Enden auf dem Außen- bzw. Innenschaft des Katheters befestigt und für den undilatierten Zustand in Längsfalten gelegt, so tragen die eine deutlich größere Wandstärke aufweisenden Enden und Konen des Ballons deutlich stärker auf als der sehr dünnwandige Zylindermantel des Ballons. Das gefaltete Ballonprofil ist also an den die Ballonschultern bildenden Konen um die Befestigungszonen am größten. Entsprechend ist auch die Steifigkeit des Ballons dort am stärksten ausgebildet. Damit behindern diese stark auftragenden Endbereiche des Ballons ein Einführen des Katheters in enge Gefäße. Die Steifigkeit der in Falten gelegten Ballonkonen erschwert darüber hinaus das Führen des Ballonkatheters um enge Krümmungen oder Abzweigungen von Gefäßen. Schließlich ist es bei der Herstellung des Ballonkatheters selbst schwierig, die eine höhere Wandstärke aufweisenden Konenbereiche zu falten.

Die vorstehend erwähnte US 5,522,882 A offenbart ein Stent-Positionierungs-System mit einem Katheter, bei dem der dilatierbare Ballon stufenförmig verlaufende Enden aufweist. Damit sollen axial vor und nach dem auf dem Katheter positionierten Stent keine Konenabschnitte des Ballons vorhanden sein. Dies wird durch hülsenartige Aufsatzstücke erreicht, die auf den Ballon-Endbereichen unmittelbar an den Stent angrenzen, so dass beim Dilatieren des Ballons sich die Konen im Wesentlichen als radiale Ringstufen ausprägen.

Die Problematik der unterschiedlichen Wandstärken und der Faltenlegung bei herkömmlichen Ballons ist in dieser Druckschrift nicht angesprochen.

Die US 7,217,278 B2 lehrt eine aufwändige Nachbearbeitung von Ballonrohlingen, indem nach dem Aufblasen im Bereich der Konen und dickwandigen Enden Wandmaterial zur Wandstärkenverringerung mechanisch abgetragen wird. Hierzu muss besonders darauf geachtet werden, dass dieser Bearbeitungsschritt unterhalb der Glasübergangstemperatur des thermoplastischen Kunststoffmaterials vorgenommen wird. Insgesamt ist also die Herstellung solcher Ballons, wie sie aus US 7,217,278 B2 bekannt sind, herstellungstechnisch aufwändig.

Die Patentannmeldung EP 2149387 A1 beschreibt einen Ballonkatheter mit einem am proximalen Ende des Ballonkatheters angebrachten dilatierbaren Ballon, der in seinem undilatierten Zustand in Längsfalten gelegt ist. Die hier offenbarten Ausführungsbeispiele beschränken sich auf das distale Ende des Ballons.

Die Patentschrift GB 2046096 A offenbart einen Ballonkatheter, bei dem zum Beispiel mit Hilfe eines Stützungsdrahtes der gesamte Dilatationsballon auf den Katheter aufgedreht werden kann.

Im US-Patent US 5,108,525 wird ein Verfahren geschützt, bei dem der Ballon mit Hilfe eines Schrumpfschlauches mit einem inneren Element verbunden wird.

In keiner der beiden letztgenannten Schriften wird auf die Problematik der Wandstärken, insbesondere an den Konen der Ballone eingegangen.

Der Erfindung liegt die Aufgabe zugrunde, einen Ballonkatheter anzugeben, bei dem auf konstruktiv einfache Weise die beim Stand der Technik vorhandenen Verdickungen und Versteifungen aufgrund der in Längsfalten gelegten Konen vermieden und gleichzeitig eine drastisch erhöhte Berststabilität der Ballonanordnung bei deren Dilatation erzielt werden.

Diese Aufgabe wird durch die Merkmale des Anspruches 1 gelöst. Das Lösungsprinzip sieht dabei zum Einen vor, den Ballon zumindest an einem Ende konenlos auszuführen, so dass er über seine Gesamtlänge eine unverändert dünne Wandstärke aufweist. In den Befestigungszonen ist der Ballon fluiddicht auf dem distalen Endbereich des Innenteils befestigt, so dass sich keinerlei konenbedingte Verdickung oder Versteifung am distalen Ende des Katheters zeigt.

Ferner werden dabei die Enden des dilatierbaren Ballons in Verdrillungen im Bereich vor den Befestigungszonen des Ballons gelegt. Damit wird die Verbindung des Ballons zum jeweiligen Schaft hin vor sogenannter schälender Belastung geschützt und die Berstempfindlichkeit des Ballons bereits grundsätzlich verbessert.

Diese Lösungsansätze sind bereits in der älteren, nachveröffentlichten Anmeldung EP 2 149 387 der Anmelderin beschrieben.

Zum anderen ist es als wesentliche Maßnahme der vorliegenden Erfindung vorgesehen, einen zweiten konenlosen Ballon über den ersten Ballon zu legen, wobei dieser zweite Ballon ebenfalls mit jeweils einer Verdrillung an seinen beiden Enden vor der Befestigungszone an Außenschaft bzw. Innenteil versehen ist. Die Verdrillungen des zweiten äußeren Ballons sind dabei den Verdrillungen des ersten darunterliegenden Ballons jeweils entgegengerichtet.

Die Funktionsweise dieser erfindungsgemäßen Maßnahme ist wie folgt zu erläutern. Durch das Verdrillen der Ballonenden wird grundsätzlich beim Dilatieren des Ballons ein Drehmoment auf die Ballonhülle erzeugt, das versucht, die Verdrillung aufzuheben. Dieser Mechanismus ist vergleichbar mit dem Aufziehen eines Bonbon-Verpackungspapiers an den beiden verdrillten Enden. Durch die übereinander liegenden, entgegen gesetzt gerichteten Verdrillungen der beiden Ballonhüllen werden nun die aufgrund des entgegen gesetzten Verdrillungssinnes auch gegenläufigen Öffnungsmomente an einem Ballonende an Ort und Stelle aufgehoben. Die Verdrillungen bleiben auch beim Dilatieren der Ballonanordnung durch Einbringen eines unter Druck stehenden Fluids erhalten. Gleichzeitig wirken sie als Einschnürung, aufgrund derer sich die beiden übereinander liegenden Ballonwandungen verstärkt auf die unter der Verdrillung liegende Struktur abstützen. Damit werden die Befestigungszonen der beiden Ballons vor schälender Belastung geschützt, die Berstfestigkeit der Ballonanordnung erhöht sich dadurch weiter erheblich. Schließlich wird dieser Effekt noch durch die Ineinanderschachtelung zweier Ballonhüllen weiter verstärkt. Damit ergibt sich insgesamt die drastische Erhöhung der Berstfähigkeit im Sinne der erfindungsgemäßen Aufgabe.

Erkennbar weist ein solcher Ballon ferner trotz Verdrillung an den Enden auf seiner gesamten Länge vergleichsweise wenig Verdickung im Durchmesser und keinerlei Versteifung durch größere Wandstärken auf. Ein solcher Ballonkatheter lässt sich also mit seinem distalen, mit dem Ballon versehenen Ende durch sehr enge Gefäße und um enge Biegungen in einem Gefäßsystem problemlos führen.

In den abhängigen Ansprüchen sind bevorzugte Ausführungsformen des Ballonkatheters angegeben, deren Merkmale, Einzelheiten und Vorteile in der nachfolgenden Beschreibung anhand der beigefügten Zeichnungen näher erläutert werden. Es zeigen:

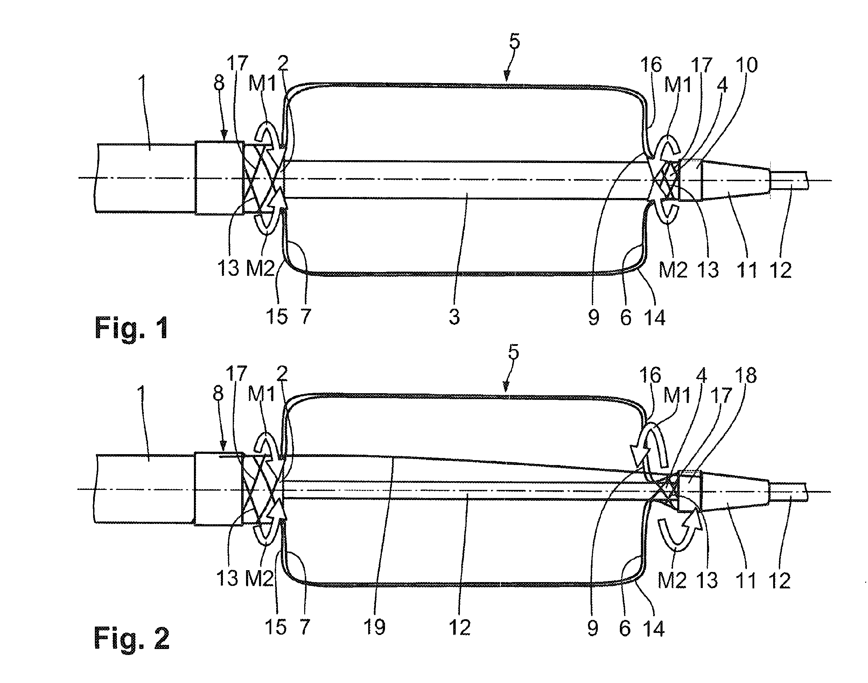

- Fig. 1 und 2: schematische Längsschnitte des distalen Endbereiches eines Ballonkatheters in zwei verschiedenen Ausführungsformen in dilatiertem Zustand.

Anhand der Fig. 1 wird der Aufbau eines Ballonkatheters in einer ersten Ausführungsform näher erläutert. Ausgegangen wird dabei von einem im Wesentlichen zweiteiligen Katheterschaft, gebildet aus einem röhrenförmigen Außenschaft 1 mit einem distalen Ende 2 und einem gegenüber dessen Innendurchmesser im Außendurchmesser deutlich kleineren Innenschaft 3, dessen distales Ende 4 um mindestens die Länge der Ballonanordnung 5 über das distale Ende 2 des Außenschaftes 1 hinaussteht. Zwischen Außen- und Innenschaft 1, 3 ist eine ringförmige Fluidleitung (nicht dargestellt) gebildet, über die ein entsprechendes Fluid unter Druck in die Ballonanordnung 5 geleitet werden kann.

Die Ballonanordnung 5 besteht aus einem inneren Ballon 6, der mit seinem proximalen Ende 7 in einer Befestigungszone 8 vor dem distalen Ende 2 des Außenschafts 1 beispielsweise durch Verschweißen oder Verkleben druckdicht fixiert ist. Sein distales Ende 9 ist analog dazu auf dem distalen Ende 4 des Innenschafts 2 in einer entsprechenden Befestigungszone 10 durch Verschweißen oder Verkleben druckdicht fixiert. Die Befestigungszone 10 liegt dabei proximal vor dem kegelförmig ausgestalteten Endstück 11 des Innenschafts 3. Dieser weist ferner ein (nicht dargestelltes) Lumen für einen Führungsdraht 12 auf.

Wie in Fig. 1 angedeutet ist, sind die beiden Enden 7, 9 des inneren Ballons 6 jeweils vor den Befestigungszonen 8, 10 in einer durch schräge Linien angedeuteten Verdrillung 13 gelegt.

Über den inneren Ballon 6 ist ein zweiter äußerer Ballon 14 angeordnet, der wiederum mit seinem proximalen Ende 15 in der Befestigungszone 8 entsprechend durch Verschweißen oder Verkleben druckdicht festgelegt ist. Auch das distale Ende 16 dieses äußeren Ballons 14 ist in der zweiten Befestigungszone 10 am Innenschaft 3 festgelegt.

Wie durch die gegenläufig zur Verdrillung 13 laufenden Schräglinien angedeutet ist, ist der äußere Ballon 14 wiederum durch eine gegenüber den Verdrillungen 13 entgegen gerichtete Verdrillung 17 vor den beiden Befestigungszonen 8, 10 verdrillt konfiguriert.

In dem in Fig. 1 nicht dargestellten zusammengefalteten Zustand sind die Ballons 6, 14 in Längsfalten eng an den Innenschaft gelegt, sodass die Ballonanordnung 5 im Durchmesser eng bleibt und nicht wesentlich über die Durchmesserdimension des Außenschafts 1 radial hinausragt.

Wird die Ballonanordnung 5 gemäß Fig. 1 durch Einbringen eines Druckfluids über die nicht dargestellte Ringleitung zwischen Innen- und Außenschaft 3, 1 in den in Fig. 1 gezeigten dilatierten Zustand übergeführt, werden durch die gegenläufigen Verdrillungen 13, 17 der beiden Ballone 6, 14 jeweils gegenläufige Drehmomente M1, M2 erzeugt, die sich gegenseitig aufheben. Gleichzeitig werden die Ballone 6, 14 im Bereich der Verdrillungen 13, 17 dadurch zusammengezogen und legen sich kraftschlüssig auf den Außen- bzw. Innenschaft 1, 3. Damit werden die Befestigungszonen 8, 10 von den durch das Dilatieren der Ballonanordnung 5 auftretenden, schälenden Kräften entlastet, insgesamt ergibt sich also eine extrem berstfeste Ballonanordnung 5.

Anzumerken ist, dass die verdrillte Anbindung der Fig.1 auch an nur einem Ballonende - wahlweise distal oder proximal - ausgeführt werden kann, indem z. B. das zweite Ballonende wie ein üblicher Ballonkonus aussieht.

Der vorstehend beschriebene "Strangulationseffekt" beim Dilatieren der Ballonanordnung 5 durch das Zusammenziehen der Ballone 6, 14 im Bereich ihrer Verdrillungen 13, 17 eröffnet auch die Möglichkeit, völlig neue Ballonkatheter-Konstruktionen umzusetzen. Ein mögliches, völlig von bisherigen Kathetern mit Innen- und Außenschaft abweichendes Katheter-Design ist in Fig. 2 gezeigt. Dieser Ballonkatheter weist lediglich einen Außenschaft 1 auf, der nur mit einem Lumen für den Führungsdraht 12 und einer Leitung für das Druckfluid zum Dilatieren der Ballonanordnung 5 versehen ist. Statt eines Innenschafts weist der Ballonkatheter gemäß Fig. 2 lediglich das Endstück 11 als eine Art Innenteil auf, auf die die beiden Ballone 6, 14 mit ihren distalen Enden 9, 16 in einer Befestigungszone 18 durch Verschweißen oder Verkleben fixiert sind. Vor dieser Befestigungszone 18 sind die beiden Ballone 6, 14 wieder in gegenläufige Verdrillungen 13, 17 gelegt, wobei in diesem Bereich innerhalb der Ballone keine Strukturteile des Endstücks 11 mehr liegen.

Die proximalen Enden 7, 15 sind analog dem Ausführungsbeispiel gemäß Fig. 1 in der Befestigungszone 8 am Außenschaft 1 durch Verschweißen oder Verkleben druckdicht festgelegt und dort ebenfalls mit den gegenläufigen Verdrillungen 13, 17 versehen.

Schematisch ist in Fig. 2 schließlich eine als Einzeldraht aus Nitinol ausgebildete Verbindungsstrebe zwischen dem distalen Ende 2 des Außenschafts 1 und dem Endstück 11 vorgesehen. Es kann auch eine Mehrfach-Drahtanordnung oder eine Art Drahtkorsett-Anordnung vorgesehen sein. Diese Verbindungsstrebe 19 läuft außerhalb der Ballonanordnung 5 im Wesentlichen parallel zur Längsachse des Ballonkatheters.

Bei dem Ballonkatheter ohne Innenschaft gemäß Fig. 2 erfolgt die Abdichtung des Balloninnenraums während des Dilatierens, indem die Ballonanordnung 5 im Bereich der distalen Verdrillungen 13, 17 durch den beschriebenen Einschnürungs- oder Strangulierungseffekt direkt auf den Führungsdraht 12 abdichtet, wobei wiederum durch die Verdrillungen 13, 17 die gegenseitige Aufhebung der Momente M1, M2 vor den beiden Befestigungszonen 8, 18 erfolgt.

Insgesamt kann der in Fig. 2 gezeigte Ballonkatheter zuverlässig entlang des Führungsdrahts 12 geschoben werden, weist also eine ausreichende "Pushability" auf. Ferner kann durch die Verbindungsstrebe(n) beim Dilatieren der Ballonanordnung 5 eine sogenannte "Scoring-Funktion" erzielt werden, das heißt, dass Stenosen durch die drahtförmigen Verbindungsstreben quasi aufgebrochen werden können.

Dadurch, dass kein Innenschaft im Ballon liegt und der Führungsdraht frei durch den Ballon läuft, kann der Ballonkatheter in der in Fig. 2 dargestellten Lösung mit einem deutlich kleineren Profil gefertigt werden.

Der in Fig. 2 gezeigte Ballonkatheter kann schließlich zur Freisetzung von Stoffen, wie Kontrastmitteln bei Diagnosekathetern benützt werden, die nach dem Zurückziehen des Führungsdrahts 12 über dessen Lumen durch den Katheter und die Öffnung im Endstück 11 ausgebracht werden können.

Der Außenschaft kann abweichend von den beiden vorgestellten Varianten (Fig.1 und Fig. 2) auch stumpf mit dem verdrillten proximalen Ende des Ballons verschweißt werden (ohne Abbildung). Dann wird das verdrillte Ballonende sich unter Innendruck zusammenziehen und die Deflatierbarkeit stark einschränken oder sogar ganz ausschalten. Um dieses zu verhindern, muss mindestens eine Kapillare zwischen dem verdrilltem Ende und dem Innenteil konstruktiv vorgesehen werden, die den Balloninnendruck durch einen Leckstrom wieder soweit abbaut, dass sich das verdrillte Ballonende wieder öffnen kann. Diese Kapillare kann z.B. durch eine Rille im Innenschaft erzeugt werden.

Ein weiterer Anwendungsfall für die beiden vorstehenden Ballonkatheter mit unter Druck selbstdichtenden Ballons sind beispielsweise der Bau aufblasbarer Implantate, die Stütz- oder Dichtfunktionen übernehmen.

## Patentansprüche

1. Ballonkatheter, umfassend
- einen Außenschaft (1) mit einem distalen Ende (2) und einer Fluidleitung,
- ein distal vor dessen distalem Ende (2) angeordnetes Innenteil (3, 11), sowie
- einen unter Einfluss eines durch die Fluidleitung unter Druck eingebrachten Fluids dilatierbaren Ballon (6), der
= mit einer ersten Befestigungszone (8) an seinem proximalen Ende (7) fluiddicht auf dem distalen Endbereich (2) des Außenschaftes (1), und
= mit einer zweiten Befestigungszone (10) an seinem distalen Ende (9) fluiddicht auf dem Innenteil (3, 11) befestigt ist,
- wobei der Ballon (6) an mindestens einem seiner beiden Enden (7, 9) konenlos mit im Wesentlichen über seine Länge unverändert dünner Wandstärke ausgestaltet ist, und
- an mindestens einem seiner beiden Enden (7, 9) mit jeweils einer Verdrillung (13) vor der Befestigungszone (8, 10) versehen ist, **dadurch gekennzeichnet, dass**
- ein zweiter Ballon (14) über den ersten Ballon (6) gelegt und ebenfalls mit jeweils einer Verdrillung (17) an mindestens einem seiner beiden Enden (15, 16) vor der Befestigungszone (8, 10) versehen ist, die der darunterliegenden Verdrillung (13) des darunterliegenden ersten Ballons (6) entgegen gerichtet ist.

2. Ballonkatheter nach Anspruch 1, **dadurch gekennzeichnet, dass** die beiden Ballone (6, 14) in den Befestigungszonen (8, 10) an beiden Enden (14, 16) verschweißt oder verklebt sind.

3. Ballonkatheter nach Anspruch 1 oder 2, **dadurch gekennzeichnet, dass** die Verdrillungen (13, 17) der beiden Ballone (6, 14) sich in die Befestigungszonen (8, 10) hinein erstecken.

4. Ballonkatheter nach einem der vorgenannten Ansprüche, **dadurch gekennzeichnet, dass** das Innenteil als distal über den Außenschaft (1) hinausstehender Innenschaft (3) ausgebildet ist.

5. Ballonkatheter nach einem der vorgenannten Ansprüche, **dadurch gekennzeichnet, dass** der Innenschaft (3) in an sich bekannter Weise mit einem Führungsdrahtlumen versehen ist.

6. Ballonkatheter nach einem der Ansprüche 1 bis 3, **dadurch gekennzeichnet, dass** das Innenteil als mit Abstand distal vor dem distalen Ende (2) des Außenschaftes (1) angeordnetes Spitzenteil (11) ausgebildet ist.

7. Ballonkatheter nach Anspruch 6, **dadurch gekennzeichnet, dass** das Spitzenteil (11) ein Führungsdrahtlumen aufweist.

8. Ballonkatheter nach Anspruch 7, **dadurch gekennzeichnet, dass** die distalen Verdrillungen (13, 17) der beiden Ballone (6, 14) proximal vor dem Spitzenteil (11) angeordnet sind.

9. Ballonkatheter nach Anspruch 8, **dadurch gekennzeichnet, dass** die Ballone (6, 14) mit ihren distalen Verdrillungen (13, 17) derart ausgelegt sind, dass sie bei Druckbeaufschlagung zum Dilatieren der Ballone (6, 14) druckdicht mit dem im Führungsdrahtlumen des Spitzenteils (11) sitzenden Führungsdraht (12) abdichten.

10. Ballonkatheter nach einem der Ansprüche 6 bis 9, **dadurch gekennzeichnet, dass** das Spitzenteil (11) durch mindestens eine Verbindungsstrebe (19) mit dem distalen Ende (2) des Außenschaftes (1) verbunden ist.

11. Ballonkatheter nach Anspruch 10, **dadurch gekennzeichnet, dass** die Verbindungsstrebe (19) außerhalb der Ballone (6, 14) verläuft.

12. Ballonkatheter nach Anspruch 11, **dadurch gekennzeichnet, dass** die Verbindungsstrebe (19) als Draht ausgebildet ist.

## Claims

1. A balloon catheter, comprising
- an outer shaft (1) having a distal end (2) and a fluid line,
- an inner part (3, 11) disposed distally in front of the distal end (2) of the outer shaft, and
- a balloon (6) which can be dilated under the action of a fluid that is introduced under pressure through the fluid line and which
= is attached by means of a first attachment zone (8) at the proximal end (7) thereof to the distal end region (2) of the outer shaft (1) in a fluid-tight manner, and
= is attached by means of a second attachment zone (10) at the distal end (9) thereof to the inner part (3, 11) in a fluid-tight manner,
- wherein the balloon (6), on at least one of the two ends (7, 9) thereof, is designed in a coneless manner having a thin wall thickness that is essentially unchanged over the length thereof, and - on at least one of the two ends (7, 9) thereof, is provided with a respective twist (13) in front of the attachment zone (8, 10),
**characterized in that**
- a second balloon (14) is placed over the first balloon (6) and likewise is provided with a respective twist (17) on at least one of the two ends (15, 16) thereof in front of the attachment zone (8, 10), the twist being directed counter to the twist (13) therebeneath of the first balloon (6) located therebeneath.

2. The balloon catheter according to claim 1, **characterized in that** the two balloons (6, 14) are welded or glued to the each other in the attachment zones (8, 10) at the two ends (14, 16).

3. The balloon catheter according to claim 1 or 2, **characterized in that** the twists (13, 17) of the two balloons (6, 14) extend into the attachment zones (8, 10).

4. The balloon catheter according to any one of the preceding claims, **characterized in that** the inner part is designed as an inner shaft (3) protruding distally over the outer shaft (1).

5. The balloon catheter according to any one of the preceding claims, **characterized in that** the inner shaft (3) is provided in the known manner with a guide wire lumen.

6. The balloon catheter according to any one of claims 1 to 3, **characterized in that** the inner part is designed as a tip part (11) disposed at a distance distally in front of the distal end (2) of the outer shaft (1).

7. The balloon catheter according to claim 6, **characterized in that** the tip part (11) comprises a guide wire lumen.

8. The balloon catheter according to claim 7, **characterized in that** the distal twists (13, 17) of the two balloons (6, 14) are disposed proximally in front of the tip part (11).

9. The balloon catheter according to claim 8, **characterized in that** the balloons (6, 14), together with the distal twists (13, 17) thereof, are designed such that they seal in a pressure-tight manner with the guide wire (12) seated in the guide wire lumen of the tip part (11) when they are pressurized to dilate the balloons (6, 14).

10. The balloon catheter according to any one of claims 6 to 9, **characterized in that** the tip part (11) is connected to the distal end (2) of the outer shaft (1) by at least one connecting strut (19).

11. The balloon catheter according to claim 10, **characterized in that** the connecting strut (19) runs outside the balloons (6, 14).

12. The balloon catheter according to claim 11, **characterized in that** the connecting strut (19) is designed as a wire.

## Revendications

1. Cathéter à ballonnet, comprenant
- un manche externe (1) ayant une extrémité distale (2) et une conduite de fluide,
- une partie interne (3, 11) disposée de manière distale à l'avant de l'extrémité distale (2) du manche externe, et
- un ballonnet (6) qui peut être dilaté sous l'action d'un fluide qui est introduit sous pression à travers la conduite de fluide et qui :
= est attaché au moyen d'une première zone de fixation (8) à l'extrémité proximale (7) de celui-ci à la région d'extrémité distale (2) du manche externe (1) d'une manière étanche aux fluides, et
= est attaché au moyen d'une seconde zone de fixation (10) à l'extrémité distale (9) de celui-ci à la partie interne (3, 11) d'une manière étanche aux fluides,
- dans lequel le ballonnet (6), sur au moins l'une de ses deux extrémités (7, 9), est conçu d'une manière sans cône ayant une fine épaisseur de paroi qui est essentiellement inchangée le long de sa longueur, et
- sur au moins l'une de ses deux extrémités (7, 9), comporte une torsion respective (13) à l'avant de la zone de fixation (8, 10),
**caractérisé par le fait que**
- un second ballonnet (14) est placé sur le premier ballonnet (6) et, de la même manière, comporte une torsion respective (17) sur au moins l'une de ses deux extrémités (15, 16) à l'avant de la zone de fixation (8, 10), la torsion étant dirigée contre la torsion (13) sous celle-ci du premier ballonnet (6) situé en dessous de celui-ci.

2. Cathéter à ballonnet selon la revendication 1, **caractérisé par le fait que** les deux ballonnets (6, 14) sont soudés ou collés l'un à l'autre dans les zones de fixation (8, 10) aux deux extrémités (14, 16).

3. Cathéter à ballonnet selon l'une des revendications 1 ou 2, **caractérisé par le fait que** les torsions (13, 17) des deux ballonnets (6, 14) s'étendent dans les zones de fixation (8, 10).

4. Cathéter à ballonnet selon l'une quelconque des revendications précédentes, **caractérisé par le fait que** la partie interne est conçue comme un manche interne (3) faisant saillie de manière distale sur le manche externe (1).

5. Cathéter à ballonnet selon l'une quelconque des revendications précédentes, **caractérisé par le fait que** le manche interne (3) comporte d'une manière connue une lumière de fil-guide.

6. Cathéter à ballonnet selon l'une quelconque des revendications 1 à 3, **caractérisé par le fait que** la partie interne est conçue comme une partie de pointe (11) disposée à une distance de manière distale à l'avant de l'extrémité distale (2) du manche externe (1).

7. Cathéter à ballonnet selon la revendication 6, **caractérisé par le fait que** la partie de pointe (11) comprend une lumière de fil-guide.

8. Cathéter à ballonnet selon la revendication 7, **caractérisé par le fait que** les torsions distales (13, 17) des deux ballonnets (6, 14) sont disposées de manière proximale à l'avant de la partie de pointe (11).

9. Cathéter à ballonnet selon la revendication 8, **caractérisé par le fait que** les ballonnets (6, 14), conjointement avec leurs torsions distales (13, 17), sont conçus de telle sorte qu'ils se scellent d'une manière étanche à la pression avec le fil-guide (12) logé dans la lumière de fil-guide de la partie de pointe (11) lorsqu'ils sont mis sous pression pour dilater les ballonnets (6, 14).

10. Cathéter à ballonnet selon l'une quelconque des revendications 6 à 9, **caractérisé par le fait que** la partie de pointe (11) est reliée à l'extrémité distale (2) de l'axe externe (1) par au moins une entretoise de connexion (19).

11. Cathéter à ballonnet selon la revendication 10, **caractérisé par le fait que** l'entretoise de connexion (19) s'étend à l'extérieur des ballonnets (6, 14).

12. Cathéter à ballonnet selon la revendication 11, **caractérisé par le fait que** l'entretoise de connexion (19) est conçue sous la forme d'un fil.
